# EUROPÄISCHE PATENTANMELDUNG

(11) **EP 2 422 702 A1**
(43) Veröffentlichungstag der Anmeldung: **29.02.2012**
(21) Anmeldenummer: 11175495.8
(22) Anmeldetag: 27.07.2011
(51) Int. Cl.: A61B 6/10, G21F 3/00

(54) **Strahlenschutzwand für Mammographiesysteme mit integrierter Benutzeroberfläche**

(30) Priorität: 31.08.2010 DE 102010035917
(71) Anmelder: Siemens Aktiengesellschaft, 80333 Munich (DE)
(72) Erfinder: Hörnig, Mathias, 91052 Erlangen (DE)

(57) **Zusammenfassung**

Die vorliegende Erfindung betrifft ein Mammographiesystem (G) mit einer Strahlenschutzwand (S), ein Herstellungsverfahren für eine Strahlenschutzwand (S) und ein Verfahren zum Bedienen eines Mammographiesystems (G), wobei die Strahlenschutzwand (S) neben dem Strahlenschutz eine weitere Funktion hat. Die Strahlenschutzwand (S) dient zusätzlich zur Darstellung einer Benutzeroberfläche eines Steuerungsprogramms zur Steuerung des Mammographiegeräts (G) und kann darüber hinaus noch die durch das Mammographiegerät (G) erfassten Bilder anzeigen. Der Displaybereich (D) der Strahlenschutzwand (S) ist im oberen Bereich der Strahlenschutzwand (S) vorgesehen und kann darüber hinaus als Touchscreen ausgebildet sein, um mit den erfassten Berührungssignalen das Mammographiegerät (G) zu steuern.

## Beschreibung

Die vorliegende Erfindung betrifft medizinische Mammographiesysteme und bezieht sich insbesondere auf eine Strahlenschutzwand für selbige.

Digitale Mammographiesysteme nach dem Stand der Technik umfassen zum einen das Akquisitionsgerät für die Mammographie und zum anderen eine Strahlenschutzwand, um einen wirksamen Schutz gegen ionisierende Strahlung zu bieten, z.B. im elektromagnetischen Spektrum des Röntgensystems. Die Strahlenschutzwand ist aus transparentem, geschliffenem und poliertem Bleiglas hergestellt. Die Strahlenschutzwand bekannter Systeme kann in eine computerbasierte Arbeitsstation integriert sein. Die computerbasierte Arbeitsstation (acquisition workstation - AWS) umfasst in der Regel einen Computer (als lokale Workstation, mit oder ohne Netzwerkanschluss), einen Monitor und eine Tastatur, sowie bedarfsweise weitere Peripheriegeräte (zum Beispiel Maus, Drucker etc.). Die einzelnen elektronischen Elemente bzw. Geräte der Arbeitsstation sind in der Regel in einem Gestell, Rahmen und/oder in einen Träger integriert.

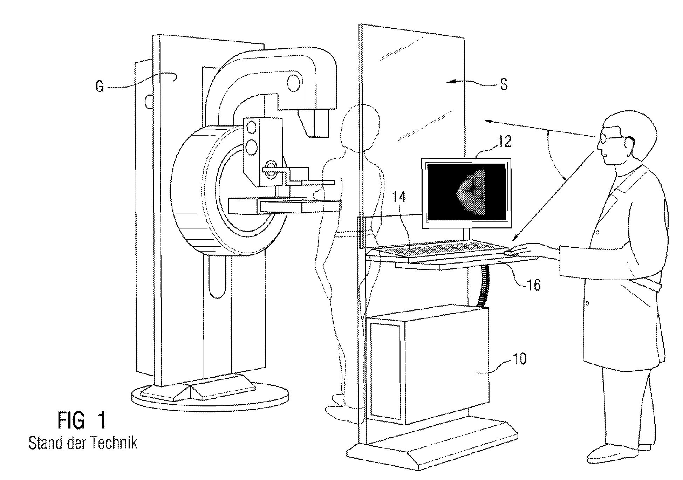

Figur 1 zeigt einen typischen Aufbau eines Mammographiesystems mit einer Arbeitsstation, in die eine Strahlenschutzwand integriert ist. Ein Anwender der Arbeitsstation (in der Regel MTRA - Medizinisch-technische Radiologieassistent/in) bedient das Mammographiesystem hinter der Strahlenschutzwand der Arbeitsstation. Dazu muss der Anwender sowohl den Patienten am Mammographiegerät als auch den Monitor der Arbeitsstation zur Steuerung des Mammographievorganges im Auge behalten. In Figur 1 ist exemplarisch ein Winkelbereich angegeben, der kennzeichnen soll, in welchem Blickwinkelbereich der Anwender bei der Steuerung des Mammographievorganges tätig sein muss. Bei Systemen nach dem Stand der Technik ist dieser relativ groß. Darüber hinaus ist es für eine fehlerfreie Durchführung des medizinischen Untersuchungsvorganges notwendig, dass der Anwender an der Arbeitsstation vorzugsweise ununterbrochen den Patienten am Mammographiegerät beobachten kann. Gleichzeitig und zusätzlich muss er aber den Computer der Arbeitsstation zur Steuerung des Mammographievorganges bedienen, was ihn aber von der ständigen Beobachtung des Patienten ablenkt. Die Ablenkung ist insbesondere dann stärker, wenn der Anwender für die Bedienung eine andere Position einnehmen und den Blickwinkel stark verändern muss.

Die vorliegende Erfindung hat sich deshalb zur Aufgabe gesetzt, eine verbesserte Strahlenschutzwand für medizintechnische Geräte zu schaffen. Insbesondere soll die Sicherheit bei der Bedienung des Mammographiesystems erhöht und die Bedienung für den Anwender erleichtert werden. Da bisherige strahlengeschützte Arbeitsstationen durch die Vielzahl der integrierten elektronischen Elemente in der Regel sehr schwer und sperrig sind, hat sich die vorliegende Erfindung darüber hinaus zum Ziel gesetzt, eine schlankere und leichter handhabbare strahlengeschützte Arbeitsstation bereitzustellen, die insbesondere leichter im Untersuchungsraum bewegt werden kann. Ferner sollen die bisher als einzelnes, separates Bauteil vorgesehenen elektronischen Komponenten, wie insbesondere der Monitor und bedarfsweise auch die Tastatur, in die Strahlenschutzwand integriert werden, so dass auf diese verzichtet werden kann.

Die Aufgabe wird durch die beiliegenden nebengeordneten Patentansprüche gelöst, insbesondere durch ein Strahlenschutzsystem, durch eine Strahlenschutzwand, durch ein Verfahren zur Herstellung einer Strahlenschutzwand und durch ein Verfahren zur Bedienung eines medizintechnischen Gerätes.

Ein wichtiger Kerngedanke der vorliegenden Erfindung ist darin zu sehen, eine Strahlenschutzwand in ihrer Funktion zu erweitern, sodass sie nicht nur zum Strahlenschutz dient, sondern zusätzlich auch noch eine Display- und Steuerfunktion umfasst.

Dazu schlägt die Erfindung vor, eine Strahlenschutzwand bereitzustellen, die einen Strahlenschutzbereich aufweist, der aus einem Strahlenschutzmaterial hergestellt ist und wobei die Strahlenschutzwand neben dem Strahlenschutz eine weitere Funktionalität hat, nämlich eine Displayfunktionalität zur Darstellung von digitalen Inhalten, und insbesondere einer Benutzeroberfläche oder eines Ausschnittes der Benutzeroberfläche eines Gerätesteuerungsprogramms. Darüber hinaus kann die Strahlenschutzwand, insbesondere der Displaybereich, auch als Touchscreen ausgebildet sein, um das Gerät sozusagen auf oder an der Strahlenschutzwand steuern zu können.

Im Folgenden sollen die, im Rahmen der vorliegenden Anmeldung verwendeten Begrifflichkeiten näher erläutert werden.

Die Strahlenschutzwand dient zur Verwendung bei technischen Geräten, die ionisierende Strahlung emittieren. Eine bevorzugte Ausführungsform der vorliegenden Erfindung bezieht sich auf medizintechnische Anwendungen. Alternativ kann die Erfindung jedoch auch auf beliebige andere technische Anwendungen bezogen sein (zum Beispiel Produktionsherstellung von Geräten mittels röntgen-basierter Verfahren; Schutzsysteme bei der Durchleuchtung von Objekten oder Körpern, wie zum Beispiel bei Flughafenkontrollsystemen oder andere technische Systeme, die einen Strahlenschutz erfordern). Im Bereich der Medizintechnik bestehen Anwendungen für Strahlenschutzwände im Bereich der Kardiologie, der Radiologie, wie der Computertomographie, der Mammographie, Urologie oder Chirurgie. Die erfindungsgemäße Strahlenschutzwand kennzeichnet sich durch eine hohe Schutzwirkung gegen ionisierende Strahlung im elektromagnetischen Frequenzbereich und durch eine zuschaltbare Displayfunktion, die vorzugsweise an- und abschaltbar ist. Die Displayfunktion bezieht sich auf die Darstellung eines Displays. Üblicherweise wird auf dem Display eine Oberfläche eines angeschlossenen Computers dargestellt.

Je nach Anwendung bzw. je nach medizintechnischem Gerät kann die Strahlenschutzwand fest in eine Abtrennwand (Mauer oder Türe etc.) integriert sein. Alternativ kann sie in eine mobile, computer-basierte Steuerkonsole integriert sein. Weitere Ausführungsformen betreffen die Verwendung der Strahlenschutzwand als einzelnes Element oder die Integration der Strahlenschutzwand in weitere Elemente eines umfassenden technischen Systems.

In einer bevorzugten Ausführungsform ist das medizintechnische Gerät ein digitales Mammographiegerät. Ein solches Gerät wird in der Regel über eine computer-basierte Steuerkonsole gesteuert. Insbesondere werden hier die Bewegung der Kompressionsflächen in Bezug auf den Detektor gesteuert, das Einstellen des Sichtfeldes (field of view - FOV), der Grad der Kompression, das Auslösen der Röntgenstrahlung etc. Die Steuerung des Mammographiegerätes übernimmt eine Anwendungsperson, in der Regel eine MTRA, die sich entweder in einem separaten Steuerungsraum befindet, der durch eine Strahlenschutzwand vom Untersuchungsraum abgetrennt ist oder die Bedienperson wird durch eine bewegliche Strahlenschutzwand vor der Strahlung geschützt. Dabei umfasst die Steuerkonsole einen Computer, einen Monitor, eine Tastatur und gegebenenfalls noch weitere Input/Output-Schnittstellen zur Benutzerinteraktion (zum Beispiel Maus etc.). Anstelle eines lokalen Computers kann es auch vorgesehen sein, lediglich eine Schnittstelle zu einem zentralen Server bereitzustellen. Üblicherweise werden alle elektronischen Geräte (Monitor, Tastatur, Computer etc.) als separate Elemente in einer gestellartigen Anordnung untergebracht. Die gestellartige Anordnung umfasst die Strahlenschutzwand. Alternativ dazu kann die gestellartige Anordnung auch flächig ausgebildet sein, wobei ein Bereich der Fläche die Strahlenschutzwand umfasst. Hierbei ist darauf hinzuweisen, dass Größe, Form. Material und Ausgestaltung der Strahlenschutzwand für die Erfindung beliebig konfiguriert werden können, so dass beispielsweise auch steckbare regalsystemartige Module oder Module aus Kunststoff und Glas kombinierbar sind.

Die hauptsächliche Ausführungsform betrifft Mammographiegeräte oder andere medizintechnische Geräte, die ebenfalls ionisierende Röntgenstrahlen emittieren, sodass das Strahlenschutzmaterial Bleiglas umfasst. Alternativ ist es jedoch auch möglich, die Erfindung auf andere technische Geräte anzuwenden, die ebenfalls einen Strahlenschutz erfordern (nicht notwendigerweise Röntgenstrahlen, sondern Strahlen in anderen Wellenlängen).

Ein wesentlicher Kerngedanke der Erfindung ist darin zu sehen, dass die Strahlenschutzwand zumindest einen Displaybereich aufweist; es können auch mehrere separate Displaybereiche bereitgestellt werden für gleiche oder unterschiedliche Funktionen (z.B. Display von der Oberfläche des Steuerungsprogramms und Display der erfassten Bilddaten (mit der gleichen Funktion, nämlich Display) und Touchscreen zur Benutzerinteraktion bei der Steuerung des Mammographiegerätes(mit einer unterschiedlichen Funktion, nämlich Touchscreen)). Weiterhin ist es nicht erforderlich, dass sich der Displaybereich über die gesamte Ausdehnung der Strahlenschutzwand erstreckt. Vorzugsweise befindet sich der Displaybereich im Sichtfeld eines Anwenders bei der Bedienung der Steuerkonsole. Der Displaybereich dient zur Darstellung von beliebigen, digitalen Inhalten und ist hierfür vorzugsweise kabellos oder kabelgebunden mit einer computer-basierten Instanz oder direkt mit der Steuerkonsole datentechnisch verbunden.

Üblicherweise dient der Displaybereich zur Darstellung einer Benutzeroberfläche oder eines Ausschnittes derselben des Steuerungsprogramms für das medizintechnische Gerät. Je nach Verwendung kann der Displaybereich auch zur Darstellung von weiteren Elementen dienen. Beispielsweise erweist es sich für den klinischen Ablauf besonders vorteilhaft, wenn zusätzlich zur Benutzeroberfläche des Steuerungsprogramms auch noch die mit dem Mammographiegerät erfassten Bildsequenzen dargestellt werden. Dies hat den Vorteil, dass das Bedienpersonal unmittelbar eine Rückmeldung der von ihr getätigten Einstellungen zur Steuerung des medizintechnischen Gerätes erhält und gegebenenfalls die Steuerung nochmals modifizieren kann. Insofern ist hier ein Regelungsmechanismus vorgesehen, der eine modifizierte Ansteuerung des medizintechnischen Gerätes ermöglicht.

Bei komplexen Anwendungen ist das Steuerungsprogramm, das auf der Steuerkonsole ausgeführt wird, was dazu führt, dass die zugehörige Benutzeroberfläche viele Darstellungselemente aufweist. In einer bevorzugten Ausführungsform ist es deshalb vorgesehen, dass nicht alle Darstellungselemente der Benutzeroberfläche auf den Displaybereich der Strahlenschutzwand dargestellt werden, sondern nur ausgewählte relevante Elemente. Ebenso ist es möglich, noch weitere Elemente in dem Displaybereich darzustellen, beispielsweise Referenzaufnahmen des Patienten zum Vergleich oder Standardeinstellungen zur Bedienung des Mammographiegerätes etc.

Für die Ausbildung des Displaybereichs der Strahlenschutzwand sind erfindungsgemäß mehrere Varianten vorgesehen, die auch kombiniert zur Anwendung kommen können und die auch durch weitere Techniken für Touchscreens erweitert werden können. Diese sind:
1. Die Displayfunktion des Displaybereichs wird durch Projektion der darzustellenden Daten auf den Displaybereich ausgeführt (zum Beispiel mittels eines Beamers, der ebenfalls Teil des Strahlenschutzsystems sein kann).
2. Die Displayfunktion wird durch Bereitstellen eines Touchscreenbereichs mit integrierter Sensorik auf der Strahlenschutzwand oder auf einem Displaybereich der Strahlenschutzwand bereitgestellt, wobei Schnittstellen zwischen dem Displaybereich und der computer-basierten Steuerkonsole vorgesehen sind.
   2a. Der Touchscreenbereich wird durch ein integriertes LED-Feld oder TFT-Feld mit entsprechenden Anschlüssen bzw. Schnittstellen zu einem Rechner bereitgestellt.
   2b. Der Touchscreenbereich wird durch eine dünne, transparente Beschichtung, z.B. eine Displayfolie, ausgebildet, die auf dem Displaybereich aufgebracht ist (zum Beispiel OLED, organic light emitting diode).

In allen Ausführungsformen können die Schnittstellen zwischen Displaybereich und Computer kabelgebunden oder kabellos (wireless, zum Beispiel Bluetooth, W-LAN, LAN etc.) ausgeführt sein.

Die Technologie für die Ausbildung des Touchscreens ist grundsätzlich nicht beschränkt und umfasst eine kapazitive, resistive, optische, oberflächenwellenbasierte oder infrarotlicht-basierte Technik.

Darüber hinaus ist es möglich, die Displayfunktionalität der Strahlenschutzwand wahlweise über eine Benutzerinteraktion an- und auszuschalten. Ebenso ist es möglich, hier Voreinstellungen zu treffen, sodass automatisch bei Aktivierung des Steuerungsprogramms auch eine Aktivierung der Displayfunktionalität ausgeführt wird. Die Displayfunktionalität kann bedarfsweise über ein Bestätigungssignal des Anwenders bestätigt werden, bevor sie aktiviert wird.

Vorzugsweise ist es voreingestellt, dass bei abgeschalteter Displayfunktionalität die Strahlenschutzwand im Displaybereich transparent ist, um eine freie Sicht auf das Mammographiegerät mit PatientIn zu gewähren.

Unter Bezugnahme auf die vorstehend erwähnten Möglichkeiten zur Ausbildung der Displayfunktionalität sei unter Bezugnahme auf die erste Ausführungsform mittels einer Projektion angemerkt, dass die Intensität (Helligkeit) eines Projektionsmittels (zum Beispiel Beamer) automatisch an den Transparenzgrad der Strahlenschutzwand angepasst wird, um eine möglichst optimale Beleuchtungssituation zu erhalten.

Gemäß einer weiteren vorteilhaften Ausführungsform ist es vorgesehen, dass die Strahlenschutzwand neben der Displayfunktionalität zumindest eine weitere Funktionalität, vorzugsweise eine Bedienfunktionalität, umfasst. So kann insbesondere eine virtuelle oder reale Tastatur in die Strahlenschutzwand integriert sein, über die der Benutzer Eingaben tätigen kann. Dies kann beispielsweise in Form einer Folientastatur erfolgen, wie es bei Mobilfunktelefontastaturen bekannt ist. Alternativ zur Fingerbedingung kann auch ein Bedienelement (zum Beispiel Stift) zur Benutzereingabe vorgesehen sein. Um keine weitere Umstellung für den Anwender und eine schnelle Bedienung des Systems möglich zu machen, wird in der Regel eine virtuelle oder reale QWERTY-Tastatur auf der Strahlenschutzwand bereitgestellt, die üblicherweise die bisherige physikalische Tastatur und die Maus ersetzt. Dies hat den Vorteil, dass die Steuerkonsole wesentlich schlanker ausgebildet werden kann und weniger elektronische Elemente umfasst. In der bevorzugten Ausführungsform kann die Strahlenschutzwand in der Tat lediglich als (mobile) Wand ausgebildet sein, in die die erfindungsgemäße Strahlenschutzwand mit Display- und/oder Input/Output-Funktionalität integriert ist. Die Strahlenschutzwand muss auch keinen eigenen Rechner mehr umfassen, sondern es kann hierfür lediglich eine Schnittstelle bzw. ein Schnittstellenmodul (Adapter) zu einem Netzwerk oder einem Server vorgesehen sein.

Damit die Bedienperson auch schriftliche Aufzeichnungen während des Mammographievorganges tätigen kann, hat es sich in der Praxis als sinnvoll erwiesen, die Strahlenschutzwand zusätzlich neben dem vertikal angeordneten Strahlenschutzelement auch noch zusätzlich mit einem horizontalen Ablageflächenelement auszubilden, die als Unterlage zum Schreiben oder zur Halterung von weiteren elektronischen Geräten dient (zum Beispiel der Tastatur oder der Maus, falls diese nicht in die Strahlenschutzwand integriert sind).

In der bevorzugten Ausführungsform umfasst die erfindungsgemäße Strahlenschutzwand keinen separaten Monitor mehr. Die Displayfunktionalität ist vollständig in die Strahlenschutzwand integriert, sodass das zusätzliche Bereitstellen eines Monitors überflüssig wird. Die weiteren elektronischen Elemente (wie zum Beispiel Tastatur, Rechner, Fußbedienpedal etc.) können einzeln und wahlweise ebenfalls in die Strahlenschutzwand integriert werden. Dies ist aber nicht zwingend erforderlich, sodass unterschiedlichste Ausführungsformen der Strahlenschutzwand mit jeweils integrierten Elementen vom Schutz dieser Anmeldung umfasst sein sollen.

Je nach Verwendung und Einsatzzweck kann die Größe, Form und Position des Displaybereichs auf der Strahlenschutzwand beliebig variiert werden. Gemäß einer bevorzugten Ausführungsform ist die Konfiguration und auch eine Veränderung des Displaybereichs (unter anderem hinsichtlich der vorstehend genannten Parameter, wie Größe, Position und Form) auch noch im Einsatz und insbesondere während eines Mammographievorganges bzw. bei fertig hergestellter Strahlenschutzwand möglich. Dies stellt einen wichtigen Vorteil gegenüber Systemen aus dem Stand der Technik dar, da die erfindungsgemäße Strahlenschutzwand auch noch im Einsatzort und somit nach Herstellung konfiguriert werden kann. Konfigurationsmöglichkeiten betreffen die Einstellung des Displaybereichs, insbesondere Einstellungen des Touchscreens, Einstellungen hinsichtlich der virtuellen Tastatur und Transparenzeinstellungen der Strahlenschutzwand. Insbesondere ist es vorgesehen, dass ein Transparenzgrad stufenlos einstellbar ist.

Durch die Konfigurations- bzw. Einstellungsmöglichkeiten entsteht der wesentliche Vorteil, dass die erfindungsgemäße Strahlenschutzwand auch nach deren Herstellung und somit im klinischen Einsatz an die jeweilige Anwendung angepasst werden kann. Soll beispielsweise eine erfindungsgemäße Strahlenschutzwand mit integriertem Displaybereich, die zusätzlich eine separate Tastatur und eine separate Maus aufweist, die jeweils auf einer horizontalen Ablagefläche angeordnet sind, in einem räumlich sehr begrenzten klinischen Einsatz zur Anwendung gebracht werden, so ist es möglich, die Strahlenschutzwand umzukonfigurieren, sodass die Strahlenschutzwand lediglich aus einem vertikalen Element besteht und die bisherige horizontal auskragende Ablagefläche abmontiert werden kann. Die neue Konfiguration ermöglicht es dann beispielsweise, dass die Tastatur und vorzugsweise weitere Benutzerinteraktionsschnittstellen ebenfalls in die Strahlenschutzwand integriert sind. Darüber hinaus kann als Ersatz für den lokalen Computer lediglich eine Schnittstelle zum Server oder zu einem Netzwerk bereitgestellt werden. Nach dieser Anwendung kann die Strahlenschutzwand durch wenige Handgriffe wieder auf die bekannte Form einer Strahlenschutzwand mit horizontaler Ablagefläche umgerüstet werden. Zum Zwecke der einfachen und schnellen Umrüstbarkeit sind alle Elemente der erfindungsgemäßen Strahlenschutzwand modular mit entsprechend standardisierten Schnittstellen ausgebildet, die einen schnellen Wechsel und Austausch der Elemente ermöglichen.

Um eine optimale Beleuchtungssituation in dem Untersuchungsraum herzustellen, ist es in einer bevorzugten Ausführungsform der Erfindung vorgesehen, dass die Strahlenschutzwand zusätzlich eine Schnittstelle zur Steuerung der Raumbeleuchtung umfasst. In die Strahlenschutzwand (insbesondere in dem Touchscreenbereich der Strahlenschutzwand) ist ein Aktionsfeld integriert, über das die Raumbeleuchtung geregelt, insbesondere gedimmt, werden kann. Darüber hinaus sind weitere Aktivierungsfelder in die Strahlenschutzwand integriert, über die der Anwender weitere Einstellungen treffen kann, beispielsweise die Transparenz der Strahlenschutzwand, das An-oder Abschalten der Displayfunktion, Zoommöglichkeiten für die einzelnen dargestellten Elemente bzw. Anordnungsmöglichkeiten von Icons und Elementen auf dem Displaybereich der Strahlenschutzwand. Darüber hinaus ist ein Auslöseelement vorgesehen, das den bisherigen Strahlauslöseknopf repräsentiert und zum Auslösen der Röntgenstrahlung des Mammographiegerätes bestimmt ist. Darüber hinaus ist in einer weiteren vorteilhaften Ausführungsform ein Hardware-Not-Aus-Schalter (der als virtueller Schalter auf dem Touchscreenbereich der Strahlenschutzwand ausgebildet sein kann) vorgesehen, um ein Not-Aus des Mammographiesystems auslösen zu können.

Ein besonderer Vorteil ist darin zu sehen, dass erfindungsgemäß wahlweise auf den Monitor und/oder auf eine separate Tastatur an der Arbeitsstation des Mammographiegerätes verzichtet werden kann, indem die Strahlenschutzwand einen Displaybereich und/oder einen Touchscreenbereich umfasst. Dabei können unterschiedliche, im Stand der Technik bekannte Touchscreensysteme verwendet werden, vorzugsweise auch Multi-Touch-Systeme, um eine effiziente Steuerung des Mammographievorganges durch Erfassung von mehreren gleichzeitigen Bedienungen auf dem berührungssensitiven Feld zu ermöglichen.

Die vorstehend erwähnten unterschiedlichen, alternativen Ausführungsformen des Strahlenschutzsystems bzw. der Strahlenschutzwand sind auch auf die anderen nebengeordneten Ansprüche zu übertragen. So kann insbesondere das Herstellungsverfahren für die Strahlenschutzwand und das Verfahren zur Bedienung des medizintechnischen Gerätes auf die unterschiedlichen Arten der Strahlenschutzwand bezogen sein.

Die vorstehend erwähnte Aufgabe wird weiterhin durch eine Strahlenschutzwand gelöst, die zusätzlich eine Displayfunktionalität und/oder eine Touchscreenfunktionalität umfasst, wie vorstehend beschrieben.

Eine weitere Aufgabenlösung besteht in einem Herstellungsverfahren für eine solche Strahlenschutzwand und in einem Verfahren zur Bedienung des medizintechnischen Gerätes (insbesondere des Mammographiegerätes) an der computer-basierten Steuerkonsole, die in die vorstehend erwähnte Strahlenschutzwand integriert ist.

Im Folgenden wird die Erfindung anhand der Zeichnungen unter Berücksichtigung von weiteren, alternativen Ausführungsformen mit deren Vorteilen näher beschrieben. In dieser zeigen:
- Figur 1: eine übersichtsartige Darstellung eines bekannten Mammographiesystems aus dem Stand der Technik
- Figur 2: eine übersichtsartige Darstellung eines Mammographiesystems gemäß einer bevorzugten Ausführungsform der Erfindung und
- Figur 3: eine alternative Ausführungsform der erfindungsgemäßen Strahlenschutzwand, wie sie in Figur 2 abgebildet ist.

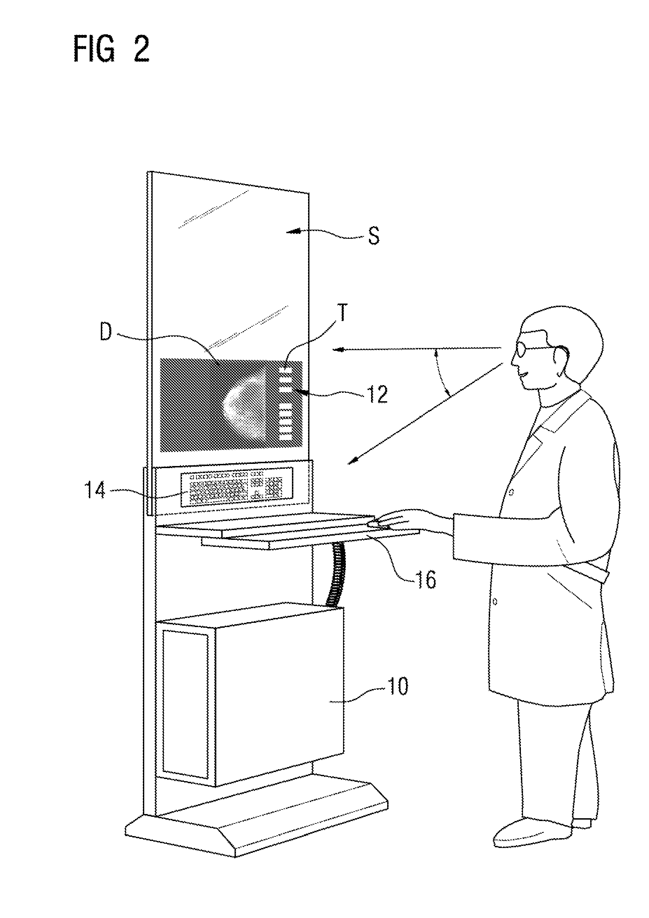

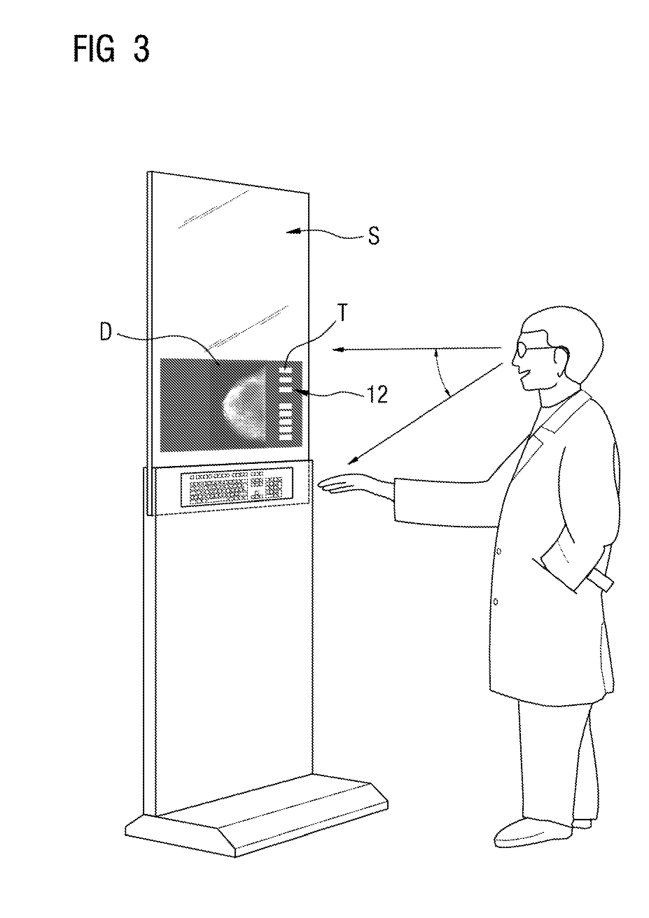

Unter Bezugnahme auf Fig. 2 wird nachstehend eine vorteilhafte Ausführungsform der vorliegenden Erfindung in Form einer Strahlenschutzwand S eines medizinischen Systems beschrieben. Die hauptsächliche Ausführungsform betrifft medizintechnische Geräte, insbesondere Mammographiegeräte G. Dieses ist exemplarisch in Fig. 1 zum Stand der Technik dargestellt. Das Mammographiegerät G steht in Datenaustausch mit einer computer-basierten Steuerkonsole, die sowohl in Figur 1, als auch in den Figuren 2 und 3 auf der rechten Seite dargestellt ist.

Die Steuerkonsole ist computer-basiert und umfasst üblicherweise einen lokalen Computer 10 und im Stand der Technik (vgl. Figur 1) eine separate Tastatur 14 und einen separaten Monitor 12, sowie eventuell weitere Input/Output-Geräte, die an der Strahlenschutzwand S angeordnet sind. Vorzugsweise ist die Strahlenschutzwand S mit einer auskragenden Halterung 16 versehen, die als Ablagefläche für den Monitor 12 und die Tastatur 14 dient.

Die Strahlenschutzwand S weist mehrere Elemente auf und ist insbesondere im oberen Bereich mit einem Strahlenschutzbereich ausgebildet, der aus einem Strahlenschutzmaterial, insbesondere Bleiglas, gefertigt ist. Im Stand der Technik ist der Strahlenschutzbereich transparent ausgebildet, um dem Bedienpersonal, das an der Steuerkonsole arbeitet, eine ungestörte Durchsicht auf den Patienten am Mammographiegerät G zu ermöglichen.

In den Figuren 2 und 3 ist die erfindungsgemäße Lösung dargestellt, bei der die Strahlenschutzwand S nicht nur die Funktion des Strahlenschutzes übernimmt, sondern darüber hinaus noch eine Displayfunktion beinhaltet. Ein Steuerungsprogramm, das auf den Computer 10 zur Steuerung des Mammographiegeräts G abläuft, wurde bisher im Stand der Technik über den Monitor 12 mit dem weiteren Ein- und Ausgabegeräten bedient. Erfindungsgemäß werden nun diese Benutzerschnittstellen zur Bedienung des Steuerungsprogramms in die Strahlenschutzwand S integriert.

In Figur 2 ist die Strahlenschutzwand S mit einem Displaybereich D zur Anzeige von digitalen Daten und einem Touchscreenbereich T zur Bedienung des Computers 10 bzw. zur Steuerung des Mammographiegerätes G mittels des Steuerungsprogramms ausgestaltet, auf dem sozusagen die Oberfläche des Steuerungsprogramms mit jeweiligen Bedienungselementen der Oberfläche abgebildet ist.

Die Strahlenschutzwand in der in Figur 2 dargestellten Ausführungsform umfasst also - ähnlich wie im Stand der Technik - die vertikale Wand, in die im oberen Bereich ein Strahlenschutzbereich integriert ist. In dem Strahlenschutzbereich ist der Displaybereich D und der Touchscreenbereich T integriert. Der Touchscreenbereich T umfasst in dem in Figur 2 dargestellten Beispiel zwei Bereiche: zum Einen einen sich vertikal erstreckenden Bereich, der neben dem Displaybereich angeordnet sein kann und graphische Aktivierungselemente zur Aktivierung der Benutzeroberfläche umfasst (wobei der in Fig. 2 und 3 dargestellte vertikale Bereich darüber hinaus auch zur Anzeige von Metainformationen zu den akquirierten Bildern bestimmt ist)und zum Anderen einen sich horizontal erstreckenden zweiten Bereich, der unterhalb des Displaybereichs angeordnet ist und eine virtuelle Tastatur repräsentiert, die die reale ersetzen soll. Darüber hinaus umfasst die Strahlenschutzwand S im Beispiel der Figur 2 zusätzlich noch die Haltevorrichtung 16 zur Ablage und den Computer 10, sowie ein Fußelement. In das Fußelement können Rollen integriert sein, um die Strahlenschutzwand mobil bzw. verfahrbar zu gestalten.

In Figur 2 sollen die Pfeile eine Blickrichtung der Bedienperson bei der Bedienung des Mammographiegerätes G kennzeichnen. Die Bedienperson muss bei der Bedienung des Mammographiegerätes G zwischen der Darstellung des Steuerungsprogramms auf dem Displaybereich D und der zu untersuchenden Person am Mammographiegerät G hin und her blicken. Die unterschiedlichen Blickwinkel, die die Person einnehmen muss, sind mit den Pfeilen in Figur 2 gekennzeichnet. Vergleicht man den Winkelbereich der erfindungsgemäßen Lösung gemäß Fig. 2 mit dem Winkelbereich nach dem Stand der Technik (Fig. 1), so wird deutlich, dass der Winkelbereich der erfindungsgemäßen Lösung wesentlich kleiner ist, sodass die Bedienperson den Blickwinkel zwischen Gerät G und Display D nur sehr geringfügig variieren muss. Die Bedienperson kann somit eine Vorzugsblickrichtung beibehalten, was insgesamt zu einer verbesserten Ergonomie und des Weiteren dazu führt, dass die Sicherheit der medizinischen Untersuchung verbessert werden kann, da eine stete und dauernde Kontrolle der Person am Mammographiegerät G gewährleistet werden kann.

Die erfindungsgemäße Strahlenschutzwand S ist modular aufgebaut, sodass deren Elemente auch im Einsatz beliebig variiert, kombiniert und neu konfiguriert werden können.

So ist es möglich, dass die Strahlenschutzwand S in einer extrem schlanken Ausführungsform, die beispielhaft in Figur 3 dargestellt ist, nur noch aus einer vertikalen Wand oder aus einem vertikalen Gestell besteht, in das die Strahlenschutzwand S integriert ist. Die horizontal auskragende Halterung 16 ist in dieser Ausführungsform nicht mehr enthalten. Ebenso fehlt der Computer 10. Es ist lediglich eine Schnittstelle an ein Netzwerk oder an einem zentralen Server vorgesehen (die in Figur 3 nicht weiter dargestellt ist). Darüber hinaus ist auch die Tastatur 14 in die Strahlenschutzwand integriert. Der Displaybereich D erstreckt sich vorzugsweise über die gesamte Breite der Strahlenschutzwand S und nahezu über die gesamte Höhe, wobei das übliche Format des Monitors 12 (Breite mal Höhe) beibehalten wurde. Der Bereich der Strahlenschutzwand S, der nicht von dem Displaybereich oder dem Touchscreenbereich T überlagert wird, kann in einer weiteren, vorteilhaften Ausführungsform der Erfindung konfiguriert werden. So kann hier insbesondere die Transparenz und die Größe dieses Bereichs gewählt werden. Voreingestellt ist, dass der verbleibende Bereich, der nicht zur Darstellung von Daten bestimmt ist, transparent verbleibt, um weiterhin den Blick auf den Patienten zu ermöglichen.

In einer weiteren Ausführungsform ist es vorgesehen, dass auf der Strahlenschutzwand in dem Displaybereich D nicht nur die Oberfläche des Steuerungsprogramms dargestellt wird, sondern zusätzlich auch noch die vom Mammographiegerät G erfassten Bildsequenzen. In welcher Größe, Form und an welcher Position die erfassten Bilddaten dargestellt werden, ist konfigurierbar.

Ein Vorteil der sich durch die erfindungsgemäße Lösung ergibt liegt darin, dass die Darstellung der Bilder wesentlich größer ausgeführt werden kann, da der Displaybereich D wesentlich größer ist als die im Stand der Technik vorgesehenen Monitore 12. Des Weiteren kann die bildliche Darstellung hinsichtlich der Beleuchtung optimal an den jeweiligen Einsatzzweck angepasst werden. Insbesondere ist es möglich, dass der Hintergrund für den Displaybereich D und/oder für den Touchscreenbereich T der Strahlenschutzwand S konfigurierbar ist und insbesondere schwarz geschaltet werden kann.

Ein wesentlicher Vorteil ist auch in der grundsätzlichen Konfigurationsmöglichkeit der erfindungsgemäßen Strahlenschutzwand S zu sehen. Vorzugsweise ist es durch den Benutzer konfigurierbar, ob die Displayfunktionalität und/oder die Touchscreenfunktionalität der Strahlenschutzwand S zugeschaltet werden soll oder nicht. Mit anderen Worten kann der Benutzer wählen, ob er mit der herkömmlichen Strahlenschutzwand S aus dem Stand der Technik mit einem separaten Monitor und einer Tastatur 14 arbeiten möchte, oder ob er die Displayfunktionalität und/oder die Touchscreenfunktionalität der Strahlenschutzwand S nutzen möchte.

Durch die Modularität der Strahlenschutzwand S ist es möglich, den Aufbau der Strahlenschutzwand S beliebig zu konfigurieren, sodass sie in der einfachsten Version lediglich aus einer vertikalen Wand S besteht (wie beispielsweise in Figur 3 dargestellt) oder sie kann in Bezug auf die bauliche Ausgestaltung so wie im Stand der Technik ausgeführt sein und einen Computer 10 und eine Halterung 16 umfassen, auf der eine Tastatur 14 abgelegt sein kann, sodass sich die erfindungsgemäße Strahlenschutzwand S in diesem Ausführungsbeispiel lediglich dadurch von dem Stand der Technik unterscheidet, dass kein separater Monitor 12 vorgesehen ist. Die Displayfunktion ist vollständig in die Strahlenschutzwand S integriert. Wichtig ist, dass in der Strahlenschutzwand S immer ein Aktivierungselement vorgesehen ist, das zur Aktivierung des medizintechnischen Gerätes bestimmt ist. Falls es sich bei dem medizintechnischen Gerät G um ein Mammographiegerät handelt, ist hier das Auslösen der Röntgenstrahlung initiierbar. Darüber hinaus umfasst die Strahlenschutzwand S ein Notaus-Element, mit dem das medizintechnische Gerät sofort Not-Aus-geschaltet werden kann. Diese Elemente (Aktivierungselement und Not-Aus-Element) können auch als zusätzliche Icons in dem Displaybereich D integriert sein. In komplexeren Ausführungsbeispielen kann die Strahlenschutzwand S zusätzlich noch weitere Funktionen umfassen und beispielsweise die Raumbeleuchtung steuern, indem eine Schnittstelle mit den Beleuchtungskörpern vorgesehen ist, um diese beispielsweise zu dimmen. Je nach Anwendung können noch weitere elektrische oder elektronische Geräte an der Strahlenschutzwand S gesteuert werden, wie beispielsweise Lautsprecher über die akustische Informationen (z.B. Positionierungshinweise für die Patientin) appliziert werden können.

Aufgrund der Modularität der Strahlenschutzwand S ist ein weiterer Vorteil darin zu sehen, dass die einzelnen Elemente der Strahlenschutzwand S auch unabhängig voneinander und sozusagen separat angeordnet und bereitgestellt werden können. Beispielsweise kann der Computer 10 der Steuerkonsole in einem anderen Raum oder an einer anderen Position untergebracht werden als das Mammographiegerät G oder als die Strahlenschutzwand S. Die jeweiligen Elemente können unabhängig voneinander bewegt werden.

In einer alternativen Ausführungsform können weitere Bedienelemente auch in einem Fußteil der Strahlenschutzwand S angeordnet sein. Hierfür eignen sich insbesondere Schaltelemente, die nur zwei Zustände annehmen können (zum Beispiel ein Strahlenauslöseelement oder der Hardware-Not-Aus-Schalter).

Je nach Ausführungsform kann der Displaybereich D lediglich zur Darstellung bzw. Anzeige von Informationen verwendet werden. Alternativ kann er auch berührungssensitiv ausgebildet werden, um als Touchscreen auch Eingaben des Benutzers umzusetzen in Steuerbefehle für das Mammographiegerät G oder andere elektronische oder elektrische Elemente (zum Beispiel die Beleuchtung).

Um die Darstellung der Daten in dem Displaybereich D zu optimieren, sind zusätzlich noch Konfigurationsmöglichkeiten vorgesehen, die in Form von weiteren Elementen auf der Strahlenschutzwand S zur Einstellung des Displays angeordnet sind (zum Beispiel wie Auflösung, Größe, Position, weitere Darstellungsdetails wie Farbe, Kontrast, Helligkeit, etc.).

### Bezugszeichenliste

- G: Medizintechnisches Gerät, insbesondere Mammographiegerät
- S: Strahlenschutzwand
- D: Displaybereich
- T: Touchscreenbereich
- 10: Computer
- 12: Monitor
- 14: Tastatur
- 16: Halterung

## Patentansprüche

1. Strahlenschutzsystem für ein medizinitechnisches Gerät (G) mit:
- Einer computer-basierten Steuerkonsole, die zum Ausführen eines Steuerungsprogramms bestimmt ist und mit
- Zumindest einer Strahlenschutzwand (S), wobei die Strahlenschutzwand (S) einen Strahlenschutzbereich aufweist, der aus einem Strahlenschutzmaterial hergestellt ist und wobei die Strahlenschutzwand (S) neben dem Strahlenschutz eine weitere Funktionalität hat, nämlich eine Displayfunktionalität zur Darstellung einer Benutzeroberfläche oder eines Ausschnittes der Benutzeroberfläche des Steuerungsprogramms.

2. Strahlenschutzsystem nach Anspruch 1, **dadurch gekennzeichnet, dass** das Strahlenschutzmaterial Bleiglas umfasst.

3. Strahlenschutzsystem nach zumindest einem der vorstehenden Ansprüche, **dadurch gekennzeichnet, dass** auf der Strahlenschutzwand (S) oder in einem Displaybereich (D) der Strahlenschutzwand (S) zumindest teilweise die Benutzeroberfläche des Steuerungsprogramms und bedarfsweise vom medizintechnischen Gerät (G) erfasste Bildsequenzen und/oder noch weitere Elemente dargestellt werden.

4. Strahlenschutzsystem nach zumindest einem der vorstehenden Ansprüche, **dadurch gekennzeichnet, dass** die Displayfunktionalität durch Projektion der Bildschirmoberfläche des Steuerungsprogramms auf die Strahlenschutzwand (S) oder auf einen Displaybereich (D) der Strahlenschutzwand (S) erfolgt.

5. Strahlenschutzsystem nach zumindest einem der vorstehenden Ansprüche, **dadurch gekennzeichnet, dass** die Displayfunktionalität durch Bereitstellen eines Touchscreenbereichs (T) mit integrierter Sensorik auf der Strahlenschutzwand (S) oder auf einem Displaybereich (D) der Strahlenschutzwand (S) erfolgt, wobei Outputschnittstellen mit einem Computer (10) der Steuerkonsole vorgesehen sind.

6. Strahlenschutzsystem nach dem vorstehenden Anspruch, **da** - **durch gekennzeichnet**, **dass** die integrierte Sensorik für den Touchscreen auf einer kapazitiven, resistiven, optischen,oberflächenwellenbasierten oder infrarotlicht-basierten Technik basiert.

7. Strahlenschutzsystem nach zumindest einem der vorstehenden Ansprüche, **dadurch gekennzeichnet, dass** die Displayfunktionalität wahlweise über eine Benutzerinteraktion aus- oder angeschaltet werden kann, wobei bei ausgeschalteter Displayfunktionalität die Strahlenschutzwand (S) transparent ist.

8. Strahlenschutzsystem nach zumindest einem der vorstehenden Ansprüche, **dadurch gekennzeichnet, dass** in die Strahlenschutzwand (S) eine Tastatur (14) integriert ist, die einem Computer (10) der Steuerkonsole zugeordnet ist und diese ersetzen kann.

9. Strahlenschutzsystem nach zumindest einem der vorstehenden Ansprüche, **dadurch gekennzeichnet, dass** ein Grad einer Transparenz der Strahlenschutzwand (S) - vorzugweise stufenlos - einstellbar ist.

10. Strahlenschutzsystem nach zumindest einem der vorstehenden Ansprüche, **dadurch gekennzeichnet, dass** sein Displaybereich (D) der Strahlenschutzwand (S) ein Aktivierungselement zur Aktivierung des medizintechnischen Geräts und/oder ein Notaus-Element als Element(e) auf einer graphischen Benutzeroberfläche des Steuerungsprogramms umfasst.

11. Strahlenschutzsystem nach zumindest einem der vorstehenden Ansprüche, **dadurch gekennzeichnet, dass** das medizintechnische Gerät (G) ein Mammographiegerät ist.

12. Strahlenschutzwand (S) zur Verwendung mit einem medizintechnischen Gerät (G), wobei die Strahlenschutzwand (S) einen Strahlenschutzbereich aufweist, der aus einem Strahlenschutzmaterial hergestellt ist und wobei die Strahlenschutzwand (S) neben dem Strahlenschutz eine weitere Funktionalität hat, nämlich eine Displayfunktionalität zur Darstellung einer Benutzeroberfläche oder eines Ausschnittes der Benutzeroberfläche des Steuerungsprogramms.

13. Verfahren zur Herstellung einer Strahlenschutzwand (S) für ein medizintechnisches Gerät (G), wobei die Strahlenschutzwand (S) einen Strahlenschutzbereich aufweist, der aus einem Strahlenschutzmaterial hergestellt ist und wobei die Strahlenschutzwand (S) neben dem Strahlenschutz eine weitere Funktionalität hat, nämlich eine Displayfunktionalität zur Darstellung einer Benutzeroberfläche oder eines Ausschnittes der Benutzeroberfläche eines Steuerungsprogramms für das medizintechnische Gerät.

14. Verfahren nach Anspruch 13, wobei in die Strahlenschutzwand (S) ein Displayfeld integriert ist, das mit einer kabelgebundenen oder kabellosen Schnittstelle zu einer computer-basierten Steuerkonsole ausgebildet ist.

15. Verfahren nach Anspruch 13 oder 14, wobei auf die Strahlenschutzwand (S) eine Displayfolie aufgebracht wird, die mit einer kabelgebundenen oder kabellosen Schnittstelle zu einer computer-basierten Steuerkonsole ausgebildet ist.

16. Verfahren zur Bedienung eines medizintechnischen Gerätes (G) an einer computer-basierten Steuerkonsole, auf der ein Steuerungsprogramm zur Steuerung des medizintechnischen Gerätes (G) ausführbar ist, wobei die Steuerkonsole zumindest eine Strahlenschutzwand (S) umfasst und wobei die Strahlenschutzwand (S) einen Strahlenschutzbereich aufweist, der aus einem Strahlenschutzmaterial hergestellt ist und wobei die Strahlenschutzwand (S) neben dem Strahlenschutz eine weitere Funktionalität hat, nämlich eine Displayfunktionalität zur Darstellung einer Benutzeroberfläche oder eines Ausschnittes der Benutzeroberfläche des Steuerungsprogramms.
